# EUROPÄISCHE PATENTSCHRIFT

(11) **EP 1 773 196 B1**
(45) Veröffentlichungstag und Bekanntmachung des Hinweises auf die Patenterteilung: **21.03.2012**
(21) Anmeldenummer: 05775073.9
(22) Anmeldetag: 27.07.2005
(51) Int. Cl.: A61B 5/151, A61B 5/157

(54) **BLUTENTNAHMESYSTEM ZUR ENTNAHME VON BLUT FÜR DIAGNOSEZWECKE**
BLOOD COLLECTION SYSTEM FOR COLLECTING BLOOD FOR DIAGNOSTIC PURPOSES
SYSTEME DE PRISE DE SANG A DES FINS DE DIAGNOSTIC

(30) Priorität: 31.07.2004 DE 102004037270
(43) Veröffentlichungstag der Anmeldung: 18.04.2007
(60) Teilanmeldung: 11001552.6
(73) Patentinhaber: Roche Diagnostics GmbH, 68305 Mannheim (DE); F. Hoffmann-La Roche AG, 4070 Basel (CH)
(72) Erfinder: SCHERER, Joerg, 4528 Zuchwil (CH); SCHMELZEIZEN-REDEKER, Guenther, 64653 Lorsch (DE); SCHMID, Wilfried, 68165 Mannheim (DE); RASCH-MENGES, Juergen, 68723 Schwetzingen (DE); PACHL, Rudolf, 67158 Ellerstadt (DE); SCHULAT, Jochen, 68167 Mannheim (DE); HOERAUF, Christian, 68723 Oftersheim (DE); DECK, Frank, 67150 Niederkirchen (DE); THOES, Bruno, 66287 Quierschied (DE); DOEPPER, Joachim, 64291 Darmstadt (DE); WINHEIM, Sven, 69181 Leimen (DE)
(86) Internationale Anmeldenummer: PCT/EP2005/008135
(87) Internationale Veröffentlichungsnummer: WO 2006/013045

(56) Entgegenhaltungen:
- WO-A-2004/060162
- DE-A1- 10 223 558
- US-A1- 2004 098 009
- US-A1- 2004 127 819
- US-B1- 6 210 420

## Beschreibung

Um für analytisch-diagnostische Zwecke eine geringe Menge Blut aus einem Körperteil (meistens dem Finger oder dem Ohrläppchen) zu entnehmen, werden Lanzetten verwendet, die zur Erzeugung einer Wunde in das entsprechende Körperteil gestochen werden. Soweit dies manuell geschieht, ist speziell trainiertes Personal erforderlich. Dennoch ist der Einstich mit erheblichem Schmerz verbunden.

Bereits seit langem werden Blutentnahmesysteme verwendet, die aus einem Stechgerät und zugehörigen, für das jeweilige Gerät speziell angepassten Lanzetten bestehen. In einem Gehäuse des Stechgerätes befindet sich ein Lanzettenantrieb, durch den eine Lanzette mechanisch in die Haut gestochen wird. Als Antriebselement für die Einstichbewegung dient hierbei üblicherweise eine Feder.

Für eine regelmäßige Überwachung bestimmter analytischer Werte des Blutes ist es jedoch häufig erforderlich, dass der Patient sich mehrfach am Tag einer Untersuchung unterziehen muss. Dies gilt insbesondere für Diabetiker, die ihren Blutzuckerspiegel häufig kontrollieren sollten, um durch Anpassung von Insulininjektionen an den Bedarf (der abhängig von der Nahrungsaufnahme, der körperlichen Tätigkeit etc. stark schwankt) ihren Blutzuckerspiegel möglichst ständig innerhalb bestimmter Sollgrenzen zu halten.

Diese Intensivtherapie setzt folglich voraus, dass die Blutentnahme mit einem möglichst geringen Schmerz verbunden ist. Mit dem Ziel, diesbezüglich eine Verbesserung zu erreichen, wurden zahlreiche unterschiedliche Blutentnahmesysteme entwickelt, die mechanische Antriebseinheiten für eine Lanzette oder Nadel beinhalten. In der heutigen Zeit sind derartigen Antriebseinheiten so hoch entwickelt, dass der Stechvorgang reproduzierbar schmerzarm ausgeführt werden kann. Bevor das Blutentnahmesystem jedoch betätigt werden kann, muss zunächst ein Antriebselement, häufig eine Feder, der Antriebseinheit in eine gespannte Position überführt werden. Beim anschließenden Auslösen des Stechvorgangs wird das Antriebselement wieder in eine entspannte Lage überführt. Die hierbei freiwerdende Kraft wird zum Antreiben der Lanzette in Einstichrichtung verwendet.

Nachteil des Standes der Technik ist es jedoch, dass der Spannvorgang eines Blutentnahmesystems bei einer mechanischen Antriebseinheit häufig kraftaufwendig ist und/ oder komplexe Handhabungsschritte durch den Benutzer erfordert. Beispielsweise wird in dem Dokument DE 10223558.9 das Spannen eines Blutentnahmesystems beschrieben, bei dem durch Drehen eines Gehäuseknopfes eine Feder in der Antriebseinheit gespannt wird. Hierfür ist der Patient gezwungen beide Hände zur Bedienung des Gerätes zu benutzen.

Häufig zeigt sich jedoch, dass derartige Handhabungsschritte von älteren oder motorisch eingeschränkten Menschen als schwierig empfunden werden. Insbesondere in der Intensivtherapie, die häufig bei älteren Menschen angewendet werden muss, ist folglich neben einem möglichst schmerzarmen Einstich, eine leichte Bedienung des Blutentnahmesystems gewünscht.

Im Stand der Technik werden Blutentnahmesysteme mit einem automatischen Antrieb beschrieben, die ein einfaches und komfortables Handling, insbesondere für Personen mit motorischen Behinderungen, ermöglichen sollen. Hierbei wird dem Benutzer der zum Teil umständliche Vorgang des Spannens der Lanzetten, sowie das anschließende Auslösen des Stechvorgangs weitestgehend erspart. Der Patient kann über einen Knopfdruck einen elektrischen Antriebsmechanismus aktivieren, ohne dass darüber hinaus weitere Handhabungsschritte notwendig sind, noch ein Kraftaufwand durch den Benutzer geleistet werden muss. Die Dokumente WO 02/100 461, WO 02/100 460 sowie WO 02/0011 und WO 02/100 251 offenbaren jeweils Blutentnahmesysteme, bei denen eine elektrische Antriebseinheit die Lanzette in Antriebsrichtung bewegt und einen Stechvorgang ausführt. Beispielsweise wird in dem Dokument WO 02/100 251 elektromagnetische Antriehselemente genannt. Über Kontrolleinheiten wird die Kraftübertragung der Antriebseinheiten auf den Lanzettenkörper gesteuert, so dass eine definierte Einstichbewegung erfolgen kann.

Ebenso wird in dem Dokument US 6,530,892 ein automatisches Blutentnahmesystem mit einer Vielzahl von Lanzetten offenbart. Analog zu dem oben genannten Stand der Technik wird die Antriebseinheit durch einen Magneten realisiert. Auch Dokument US 2004/098009 beschreibt ein System mit magaziniesten Testsheifen. Die Rotation des Magazins erfolgt durch einen Elektromotor, der zusätzlich den Antriebsmechanismus des Lanzelle mit Energie versorgen Kann.

Die beschriebenen Systeme bedienen sich jeweils elektrischer Antriebseinheiten. Eine schnelle Kraftübertragung auf die Lanzette, die die elektrische Energie in eine Bewegung des Lanzettenkörpers umwandelt, muss über zusätzliche Bauelemente verwirklicht werden. Um eine hohe Antriebsgeschwindigkeit auch bei elektrischen Antriebseinheiten realisieren zu können, werden beispielsweise Kondensatoren in eine elektrische Antriebseinheit integriert, die durch ein schnelles Entladen, die zum Einstich benötigte Energie zur Verfügung stellen. Auf diese Weise wird versucht, die Energie schnell und unmittelbar von der elektrischen Antriebseinheit auf die Lanzette zu übertragen. Obwohl elektrische Antriebseinheiten aufgrund ihrer hohen Speichedichte von > 100 Joule pro Gramm für einen Einsatz als Langzeitspeicher in Stechhilfen besonders vorteilhaft sind, zeigt es sich jedoch, dass die Entnahmegeschwindigkeit der Energie von elektrischen Antriebseineiten wegen eines gegebenen Innenwiderstandes üblicherweise auf einige 10 Joule/Sek. begrenzt ist. Mit zunehmender Entnahmegeschwindigkeit verschlechtert sich zudem der Wirkungsrad des Systems. Zur Steuerung eines definierten Bewegungsablaufes des Lanzettenkörpers sind darüber hinaus zusätzliche Maßnahmen erforderlich, die einen vibrationsarmen Einstich der Lanzette in ein Körperteil eines Patienten gewährleisten, sowie den anschließenden Rückzug der Lanzette in das Blutentnahmesystem.

Nachteil des beschriebenen Standes der Technik ist folglich, dass neben den elektrischen Antriebseinheiten aufwendige Kontrollmechanismen erforderlich sind, die einen definierten Bewegungsablauf des Lanzettenkörpers während des Einstichvorgangs steuern. Aufgrund der elektrischen Antriebseinheit können in den Systemen die bereits hinlänglich erprobter mechanische Antriebsmechanismen nicht integriert werden. Auf die Vorteile der in den letzten Jahren immer wieder neu entwickelten mechanischen Antriebseinheiten, die einen schmerzarmen Einstich aufgrund exakt definierter Bewegungsabläufe des Lanzettenkörpers erzielen, muss folglich verzichtet werden.

Um eine zu mechanischen Antriebseinheiten vergleichbare Steuerung des Stechvorgangs bei elektrischen Antrieben umzusetzen, müssen somit aufwendige, zusätzliche Maßnahmen vorgenommen werden, die sich dennoch im Vergleich zu den mechanischen Antriebseinheiten häufig als ungenügend erweisen. Die hierfür erforderlichen zusätzlichen Bauteile wie z. B. Kondensatoren und Kontrolleinheiten gestalten darüber hinaus den Aufbau einer Stechhilfe aufwendig und erhöhen deren Herstellungskosten. Des weiteren erfolgt neben der unzureichenden Steuerung des Bewegungsablaufes Häufig eine verzögerte Kraftübertragung der Antriebseinheit auf den Lanzettenkörper, so dass es zu einem verlangsamten Bewegungsablauf des Lanzettenkörpers kommt. Ilieraus resultiert jedoch wiederum eine Vergrößerung des Einstichschmerzes. Ein Vergleich von elektrischen Antriebseinheiten mit mechanischen Antriebseinheiten zeigt folglich dass elektrische Antriebseinheiten zwar zum einen eine höhere Speicherdichte aufweisen, die dem System zur Verfügung steht, zum anderen erfolgt jedoch die Entnahmegeschwindigkeit und somit die Kraftübertragung der Antriebseinheit auf den Lanzettenkörper häufig nur mangelhaft.

Es zeigt sich somit, dass Blutentnahmesysteme mit einer elektrischen Antriebseinheit nur schwer den Anforderungen bezüglich eines schmerzarmen Einstichs genügen können.

Die Verwendung einer mechanischen Antriebseinheit ist hingegen durch eine hohe Entnahmegeschwindigkeit definiert, wie sie z. B. für eine Stechbewegung einer Lanzette dringend benötigt wird. Übliche Federn liefern hier eine hohe Entnahmegeschwindigkeit von einigen tausend Joule pro Sekunde bei annähernd idealem Wirkungsgrad. Als Energiespeicher erweisen sich jedoch die mechanischen Antriebseinheiten, die häufig unter anderem in Form einer Feder realisiert sind, als ineffizient, da für eine hohe Speicherdichte große Volumina erforderlich wären. Im Vergleich zu elektrischen Antriebseinheiten besitzt z. B. eine in einer typischen mechanischen Antriebseinheit verwendeten Feder eine geringe Speicherdichte von nur ca. 150 mJ pro Gramm. Im Hinblick auf eine kompakte Bauweise des Blutentnahmesystems, wie es in der modernen Analytik geordert wird, kann folglich eine hohe Speicherdichte bei einem mechanischen Antrieb nicht umgesetzt werden. Darüber hinaus erfordert die Verwendung von mechanischen Antriebseinheiten häufig ein kompliziertes und kraftaufwendiges Handling von dem Benutzer, was wie bereits oben beschrieben als nachteilig angesehen wird.

Aus dem Dokument US2004/0127819 ist eine Antriebseinheit bekannt, bei der ein mechanischer Energiespeicher in Form einer Feder an einen Elektromotor gekoppelt ist.

In den letzten Jahren hat sich somit immer mehr gezeigt, dass ein großes Interesse an einem Blutentnahmesystem besteht, das die schwierigen und teilweise gegenläufigen Anforderungen (minimales Schmerzempfinden, einfache Bedienung, kompakte, möglichst schlanke Bauweise und einfache, kostengünstige Konstruktion) gleichzeitig möglichst weitgehend erfüllt.

Aufgabe der Erfindung ist es, die beschriebenen Nachteile des Standes der Technik zu vermeiden und ein Blutentnahmesystem bereitzustellen, dass ein einfaches Handling insbesondere für ältere oder behindert Personen zulässt, wobei ein schmerzarmer Einstich zur Blutentnahme gewährleistet werden soll. Hierbei sollte insbesondere das häufig komplizierte und kraftaufwendige Spannen eines Blutentnahmesystems vereinfacht werden.

Die erfindungsgemäße Aufgabe wird durch die unabhängigen Ansprüche gelöst. Besonders bevorzugte Ausführungsformen ergeben sich gemäß den abhängigen Ansprüchen.

Die Erfindung beinhaltet eine Stechhilfe zum Erzeugen einer Hautöffnung in einen Körperteil. Das Gehäuse der Stechhilfe weist eine Öffnung auf, aus der eine Lanzette austreten kann. Innerhalb des Gehäuses wird eine Lanzette positioniert, die über eine Antriebseinheit zum Ausführen eines Stechvorganges angetrieben wird. Die Antriebseinheit des Blutentnahmesystems beinhaltet erfindungsgemäß einen mechanischen Energiespeicher der über einen Motor mit einem elektrischen Energiespeicher verbunden werden kann, sodass Energie zum Antreiben der Lanzette bereitgestellt werden kann. Hierbei zeichnet sich ein elektrischer Energiespeicher, wie bereits beschrieben, durch eine hohe Speicherdichte aus, und kann z. B. Batterien oder Akkus beinhalten. Zur Entnahme der Energie aus dem elektrischen Energiespeicher wird ein Motor an den elektrischen sowie an den mechanischen Energiespeicher gekoppelt, so dass die dem Motor zur Verfügung gestellte, elektrische Energie in mechanische Energie umgewandelt und gespeichert werden kann. Der mechanische Energiespeicher zeichnet sich hingegen durch eine schnelle Entnahmegeschwindigkeit aus und stellt dem System die zur Ausführung eines Stechvorganges benötigte Energie innerhalb weniger Millisekunden zur Verfügung. Der Lanzettenkörper ist über einen Kopplungsmechanismus an den mechanischen Energiespeicher angekoppelt, so dass die gespeicherte Energie direkt und unmittelbar auf den Lanzettenkörper übertragen werden kann. In einer bevorzugten Ausführungsform beinhaltet der Kopplungsmechanismus zwischen mechanischen Energiespeicher und Lanzette einen mechanischen Bewegungswandler zur Steuerung des Bewegungsablaufes des Lanzettenkörpers, so dass die Lanzette eine zwangsläufig geführte Bewegung ausführt. Auf diese Weise kann die Lanzettenbewegung in oder gegen die Einstichrichtung in der Weise gesteuert werden, dass ein schmerzarmer Einstich gewährleistet wird.

Das erfindungsgemäße System ermöglicht die Kombination eines elektrischen Motors mit einem mechanischen Energiespeicher und somit mit weiteren mechanischen Bauelementen einer Antriebseinheit eines Blutentnahmesystems, so dass ein kontrollierter Bewegungsablauf des Lanzettenkörpers während des Einstichs gewährleistet werden kann. Das System kann somit zur Ankopplung des Lanzettenkörpers an den mechanischen Energiespeicher auf bereits bekannte Prinzipien von mechanischen Bauelementen, wie z. B. Steuerkulissen (s. z.B. US 6,409,740 und US 6,419,661), die zur Kopplung eine Feder an eine Lanzette verwendet werden, zurückgreifen. Auf diese Weise können die in den letzten Jahren bereits bewährten Bauelemente zur definierten Führung des Lanzettenkörpers in einem mechanischen Antrieb in das System integriert werden. Hierbei können auf aufwendige Kontrolleinheiten, wie sie bei elektrischen Antrieben erforderlich sind, verzichtet werden. Das System verfügt darüber hinaus sowohl über eine hohe Speicherdichte, als auch gleichzeitig über eine hohe Entnahmegeschwindigkeit. Das erfindungsgemäße Blutenthahmesystem zeichnet sich folglich durch die Kombination eines elektrischen Motors mit einem mechanischen Energiespeicher aus, so dass die elektrische Energie in mechanische Energie umwandelt werden kann.

Ein mechanischer Energiespeicher kann im Sinne der Erfindung auf vielfältige Weise verwirklicht werden. Vorteilhafterweise ist der mechanische Energiespeicher als Festkörper in die Antriebseinheit integriert. Ein derartiger Energiespeicher ist z. B. eine Feder, wie sie bereits bei mechanischen Antriebseinheiten Anwendung findet. Ein Spannen der Feder erfolgt dann durch einen elektrischen Motor, der die Feder zunächst komprimiert. Als Feder können beispielsweise eine Spiralfeder, Torsionsfeder oder eine Schenkelfeder etc. wie sie im Stand der Technik ebenfalls hinlänglich bekannt sind, eingesetzt werden. Der Stechvorgang kann durch Entspannen der Feder, wie bei mechanischen Antriebseinheiten, entsprechend ausgelöst werden, sowie entsprechende Kopplungsmechanismen zwischen der Feder und dem Lanzettenkörper Anwendung finden können.

Für das Spannen der Feder kann es sich unter Umständen als hilfreich erweisen, dass der Motor über eine Kupplung und/oder über ein Getriebe an den mechanischen Energiespeicher gekoppelt ist. Auf diese Weise kann problemlos das zum Spannen der Feder benötigte Drehmoment bereits von kleineren Motoren zur Verfügung gestellt werden.

Wird eine Kupplung als Verbindung zwischen Motor und mechanischem Energiespeicher verwendet, können somit auf einfache Weise Drehmomente von 30 mNm auf den mechanischen Energiespeicher übertragen werden. Als Kupplung zwischen Motor und mechanische Energiespeicher sind beispielsweise drehmoment- oder drehwinkelgesteuerte Kupplungen vorteilhaft, die gleichzeitig auf einfache Weise eine Steuerung des Motors erlauben. Als Getriebe können z. B. Kegelradgetriebe verwendet werden. Ebenso sind auch weitere im Stand der Technik bereits bekannte Getriebe- oder Kupplungsarten etc. denkbar, die eine Kopplung des Motors an den mechanischen Energiespeicher ermöglichen.

Wird eine Kupplung zur Kraftübertragung des Motors auf den mechanischen Energiespeicher, verwendet, ist es wie oben beschrieben denkbar, dass der Motor aufgrund des anliegenden Drehmomentes gesteuert wird. Hierbei wird in einer vorteilhaften Ausführungsform der Motorstrom während des Spannvorganges gemessen und mit vorgegebenen Werten verglichen. Wird beispielsweise der mechanische Energiespeicher durch eine Feder realisiert, erhöht sich das Drehmoment zum Spannen der Feder mit zunehmender Komprimierung der Feder. In Abhängigkeit von der Kompression der Feder erhöht sich folglich der Motorstrom, wobei der jeweils gemessene Wert des Motorstroms einen definierten, komprimierten Zustand der Feder wiedergibt. Anhand eines vorgegebenen Wertes des Motorstroms, kann somit dem System signalisiert werden, dass der Spannvorgang vollständig ausgeführt wurde. Überschreitet der Motorstrom einen derartigen Schwellenwert, wird der Motor gestoppt, so dass der Spannvorgang der Feder beendet wird. Eine automatische Steuerung des Motors wird folglich auf einfache Weise realisiert. Die beschriebene, vorteilhafte Ausführungsform erlaubt einen Antrieb des Blutentnahmesystems ohne dass zusätzliche Positionssensoren etc. zur Steuerung des Betriebsablaufes benötigt werden. Das Auslösen des Stechvorgangs kann anschließend auf gleiche Weise über eine Steuerung durch das anliegende Drehmoment erfolgen. Hierbei wird der Motor zunächst erneut aktiviert, so dass der Spannvorgang der Feder zunächst fortgeführt wird, bis ein zweites, vorgegebenes Drehmoment, ein zweiter Schwellenwert, erreicht wird. Mit Erreichen des vorgegebenen Drehmoments des zweiten Schwellenwertes wird nun die Verbindung zwischen dem Motor und der Feder automatisch gelöst, so dass die von der Feder gespeicherte, mechanische Energie freigegeben werden kann. Die Feder entspannt sich, wobei der Lanzettenkörper über die von der Feder freigesetzte Energie angetrieben wird. Hierbei kann die Energie über entsprechende Kopplungsmechanismen, wie sie im Stand der Technik zur Kraftübertragung zwischen Feder und Lanzettenkörper verwendet werden, definiert auf den Lanzettenkörper einwirken.

Eine analoge Steuerung des Motors ist darüber hinaus auch durch weitere Ausführungsformen denkbar, bei denen z. B. eine Steuerung der Kupplung über den Drehwinkel erfolgt.

Des Weiteren kann ein mechanischer Energiespeicher in Form eines Festkörpers ebenfalls durch eine Masse verwirklicht werden, die beispielsweise durch einen Motor in Rotation versetzt wird. Die auf diese Weise erzeugte kinetische Energie wird auf den Lanzettenkörper übertragen, so dass die Lanzette eine Einstichbewegung ausführt. Es zeigt sich folglich, dass die elektrische Energie des Motors sowohl in Form von potentieller als auch kinetischer Energie umgewandelt und durch einen mechanischen Energiespeicher gespeichert werden kann. Wird die elektrische Energie durch den mechanischen Energiespeicher als kinetische Energie gespeichert, muss anschließend auch hier eine Kopplung des Lanzettenkörpers mit dem mechanischen Energiespeicher erfolgen, so dass die gespeicherte Energie unmittelbar und verlustarm freigesetzt werden kann und in eine gezielte Bewegung des Lanzettenkörpers überführt wird. Hierfür ist bei der Verwendung einer rotierenden Masse als Energiespeicher vorteilhafterweise eine Kupplung als Kopplungsmechanismus zwischen mechanischem Energiespeicher und Lanzettenkörper vorge sehen, die die kinetische Energie der Masse wiederum auf den Lanzettenkörper oder in eine bevorzugte Ausführungsform auf einen mechanischen Bewegungsumwandler wirken lässt. Eine derartige Kupplung kann beispielsweise eine Reibrichtsperre und eine Welle wie sie im Stand der Technik hinlänglich bekannt sind enthalten. Ebenso sind Ausführungsformen mit einer Schlingfeder oder mit einer Automatikkupplung wie nachfolgend noch näher beschrieben wird, denkbar. Durch die Integration einer Kupplung zwischen der bewegten Masse und dem Lanzettenkörper wird eine hohe Entnahmegeschwindigkeit der Energie und somit eine Kupplungszeit vorzugsweise im Bereich von 1 ms ermöglicht.

Als Kopplungsmechanismen zwischen mechanischem Energiespeicher und Lanzettenkörper sind folglich neben den im Stand der Technik bereits bekannten Ausführungsformen zur Kopplung der Feder mit dem Lanzettenkörper weiterhin auch die Verwendung von Kupplungen denkbar, die eine unmittelbare Übertragung der Energie von einer bewegten Masse auf den Lanzettenkörper erlauben. Eine Kopplung zwischen mechanischem Energiespeicher und Lanzettenkörper kann somit zum einen durch einfache Mechanismen, wie sie beispielsweise für mechanischen Antriebseinheiten in US 5,318,584 beschrieben werden, realisiert werden. Zum anderen erweist sich auch der Gebrauch von Kupplungen und/oder Getrieben als vorteilhaft. Hierbei kann das Bauelement, was für die Kopplung zwischen mechanischem Energiespeicher und Lanzettenkörper verantwortlich ist, auch gleichzeitig als mechanischer Bewegungswandler fungieren oder wiederum über einen mechanischen Bewegungswandler mit dem Lanzettenkörper verbunden sein, so dass die Lanzette eine zwangsläufig geführte Bewegung ausführt. Beispielhaft sei hierfür ein Kreuzschleifgetriebe genannt, das eine Kopplung der Bauelemente ermöglicht und gleichzeitig als mechanischer Bewegungswandler dient.

Die genannten Ausführungsformen sind nur beispielhaft aufgeführt. Weitere Ausführungsformen wie sie im Stand der Technik zur Übertragung von Energie bekannt sind, sind ebenfalls denkbar sowie eine Kombination dieser Ausführungsformen mit den im Stand der Technik bekannten mechanischen Energiespeichern.

Als mechanischer Bewegungswandler zur definierten Führung des angetriebenen Lanzettenkörpers, können ebenfalls Mechanismen wie sie im Stand der Technik bei mechanischen Antriebseinheiten verwendet werden, zurückgegriffen werden. Beispielsweise sei hier das Prinzip einer Steuerkulisse (DE 10223558.9) genannt. Hierbei wird aufgrund von einer Steuerkulisse ein definierter Bewegungsablauf des Lanzettenkörpers in und entgegen der Einstichrichtung ermöglicht. Ebenso ist jedoch auch hier die Verwendung von Getrieben, z. B. Kreuzschleifgetriebe, wie oben beschrieben, möglich.

Das erfindungsgemäße System erlaubt die Integration eines elektrischen Motors in herkömmliche, mechanische Antriebseinheiten von Blutentnahmesystemen. Auf diese Weise können den hohen Anforderungen an einen schmerzarmen Einstich genügt werden, wobei gleichzeitig ein komfortables Handling insbesondere für motorisch eingeschränkte Patienten gewährleistet wird. Das erfindungsgemäße System ermöglicht dabei einen einfachen und kostengünstigen Aufbau. Erfindungsgemäß wird dabei die Ankopplung eines Motors an mechanische Antriebselemente realisiert, wie sie im Stand der Technik hinlänglich bekannt sind. Dies wird insbesondere dadurch gelöst, dass elektrische Energie durch einen Motor in mechanische Energie umgewandelt wird. Durch den Einsatz eines mechanischen Energiespeichers ist die Ankopplung an weitere mechanische Bauelemente wie z.B. einen mechanischen Bewegungswandler, wie sie im Stand der Technik für eine definierte Führung der Einstichbewegung in mechanischen Antriebseinheiten verwendet werden, möglich. Auf diese Weise ist sowohl eine hohe Entnahmegeschwindigkeit als auch ein definierter Bewegungsablauf gegeben.

Als Motor können beispielsweise elektrische Motoren (DC-Motoren, Außenläufer/Motoren oder bürstenlose Motoren (brushless) oder eine sogenannte "Memory shaped Alloy Actuator", verwendet werden. Bei dem sogenannten "Memory shaped Alloy Actuator", der im Stand der Technik auch als "Nanomuscle" bezeichnet wird, werden Einzelelemente, bestehend aus vorzugsweise hochreinen Legierungen, durch einen Strom erhitzt,so dass sich hierdurch ihre Form (Ausdehnung der jeweiligen Elemente) verändert).

Das erfindungsgemäße System erweist sich darüber hinaus als besonders vorteilhaft bei der Anwendung in integrierten Systemen, die in einem Analysesystem Vorteilhafterweise mehrere Funktionen in sich vereinigen. Derartige Systeme ersparen dem Benutzer komplexe Handhabungsschritte durch die Integration mehrerer Systemfunktionen in einem Gerät. Die Verwendung von integrierten Systemen ermöglicht dem Benutzer somit u. a. mittels eines einzigen Gerätes zunächst einen Stechvorgang auszuführen und anschließend das Blut auf einem vom System bereitgestellten Testelement aufzugeben. Eine Analyse des Testelementes erfolgt dann unmittelbar im Gerät, ohne dass der Patient zwischen verschiedenen Geräteelementen (Stechhilfen, Testelemente, Messgerät) wechseln muss. Beispielsweise wird eine Integration einer Stechhilfe in ein Messgerät in dem Dokument WO 98/24366 beschrieben. Dem Patienten wird somit ermöglicht, alle zur Analyse notwendigen Handhabungsschritte durch ein Gerät auszuführen. Es sind jedoch auch Systeme im Stand der Technik bekannt, die hiervon abweichende Arten der Integration aufweisen.

Beispiele für weniger komplexe Geräte, bei dem die Stechhilfe separat zum Messgerät gehandhabt wird, weisen eine Magazinierung von Testelementen auf, sowie eine automatische Ausgabe von Testelementen. Beispielhaft sei hier das Gerät AccuCheck Compact® der Firma Roche Diagnostics GmbH genannt. Derartig integrierte Systeme können darüber hinaus vorteilhafterweise neben einem Testelementmagazin auch Lanzettenmagazine aufweisen. Wird ein erfindungsgemäßes System mit einem integrierten System, wie beschrieben kombiniert, können somit den hohen Anforderungen an ein komfortable Handling entsprochen werden.

Bei der Integration des erfindungsgemäßen Systems kann der elektrische Motor dann in einer vorteilhaften Ausführungsform als ein kombinierter Antrieb verwendet werden. Bei einem kombinierten Antrieb im Sinne der Erfindung stellt der elektrisch betriebene Motor zum einen Energie für den mechanischen Energiespeicher wie beschrieben zur Verfügung, zum anderen kann der elektrisch betriebene Motor jedoch auch gleichzeitig oder zeitlich unabhängig davon für eine weiter Systemfunktion Energie zur Verfügung stellen. Diese Systemfunktion kann beispielsweise ein Magazintransport, Testelementtransport etc. sein.

Wird der Motor zeitlich nacheinander für verschiedene Funktionen verwendet, erweist es sich als vorteilhaft ein weiteres Getriebe und/oder Kupplung zu verwenden, die den Motor an die entsprechende Systemfunktion an- oder entkoppeln. Hierdurch kann sowohl eine räumliche als auch ein zeitlich unabhängiger Gebrauch des Motors für die jeweiligen Systemfunktionen leicht realisiert werden. Eine kompakte Bauweise von hochintegrierten Systemen wird somit möglich.

Im folgenden werden anhand der Figuren beispielhaft bevorzugte Ausführungsformen näher beschrieben.

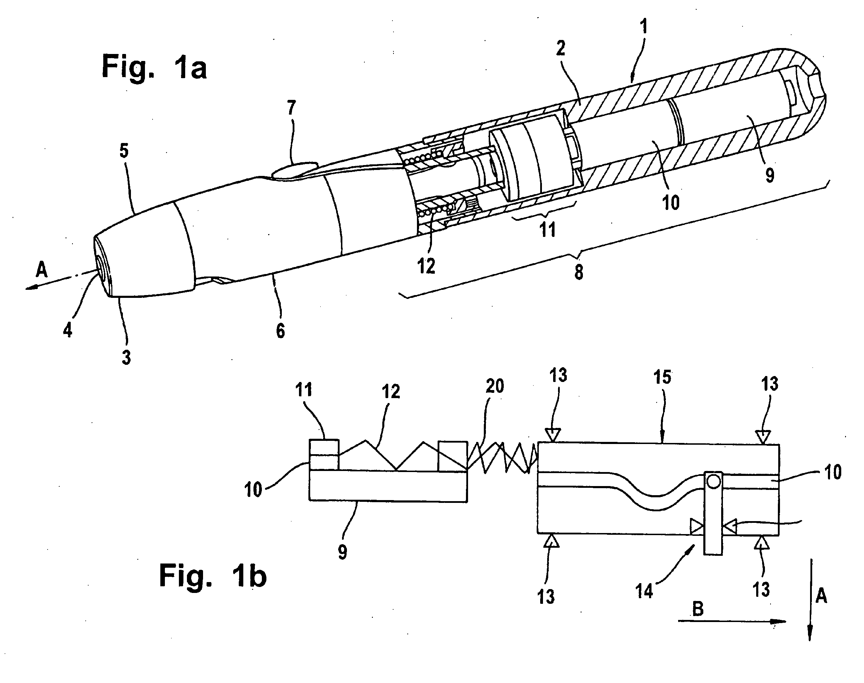

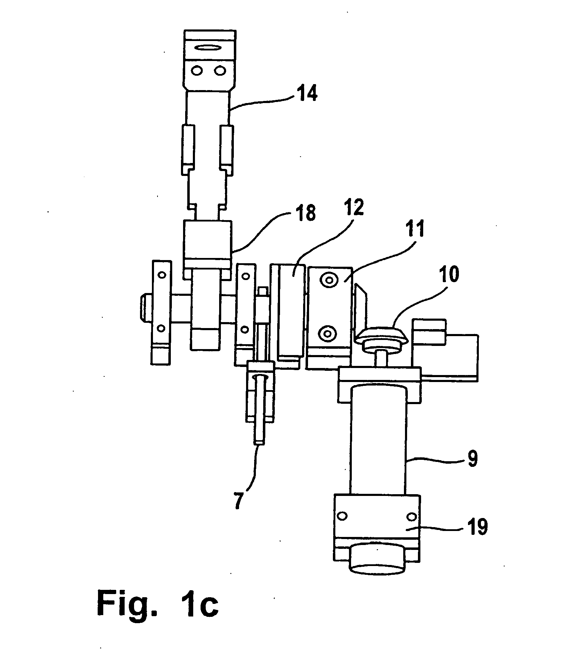

- Figur 1 a): Elektrische Stechhilfe mit einer Feder als mechanischer Energiespeicher.
- Figur 1 b): Elektrische Stechhilfe mit Kegelradgetriebe und Feder.
- Figur 1 c): Elektrische Stechhilfe mit beweglich geführter Kulisse.
- Figur 2: Rotierende Masse als mechanischer Energiespeicher mit Automatikkupplung.
- Figur 3 a): Integriertes Systems mit einem kombinierten Antrieb.

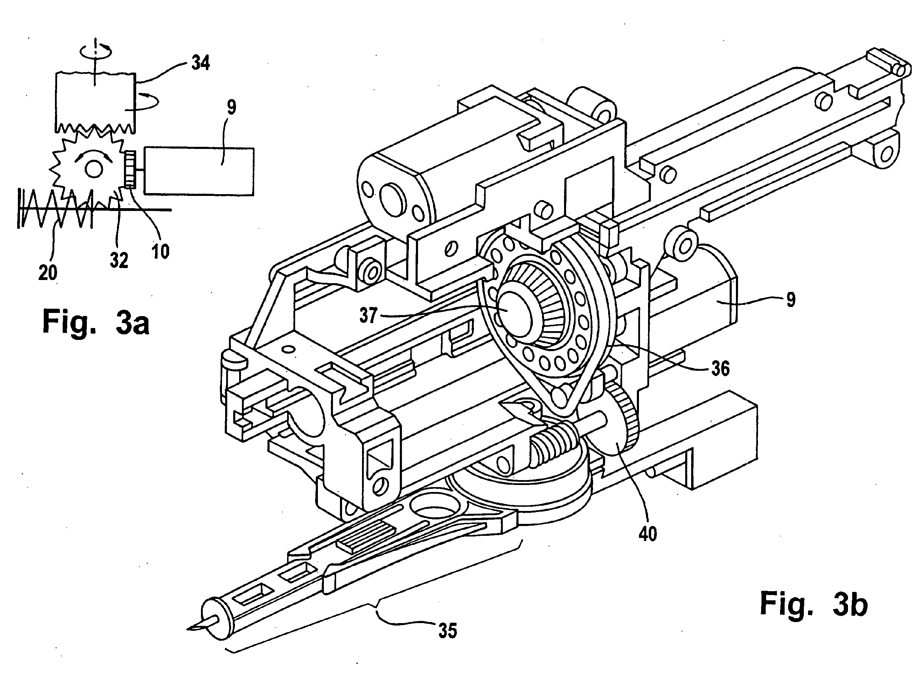

- Figur 3 b): Integriertes System mit kombiniertem Antrieb für ein Trommelmagazin.

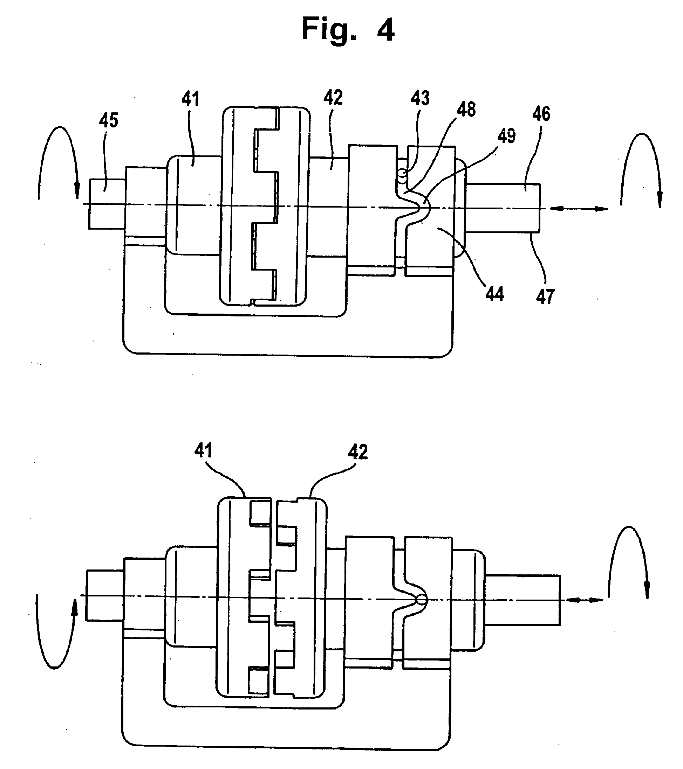

- Figur 4: System mit drehwinkelgesteuerter Kupplung.

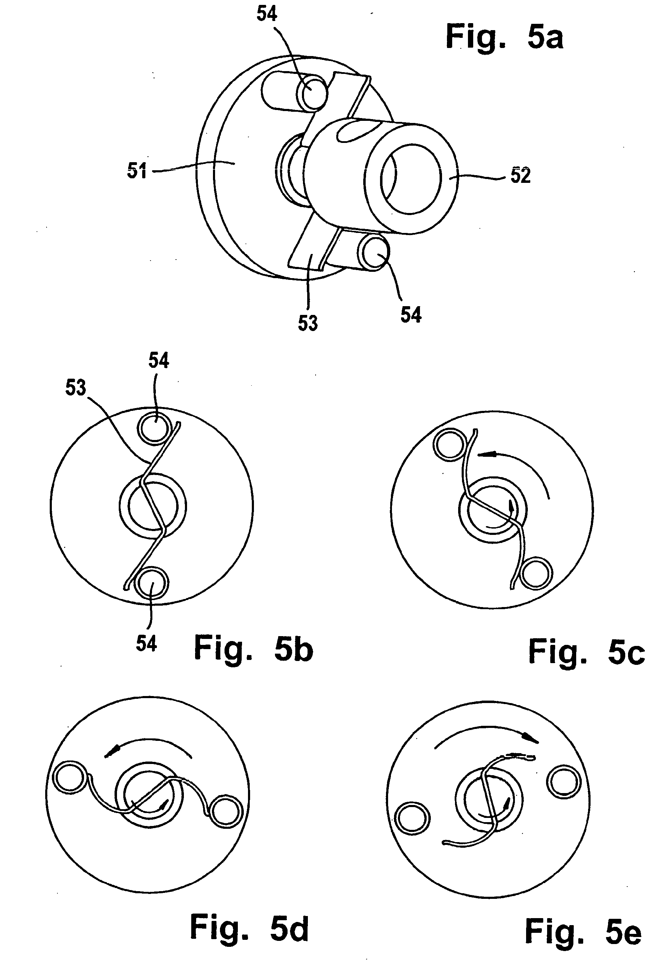

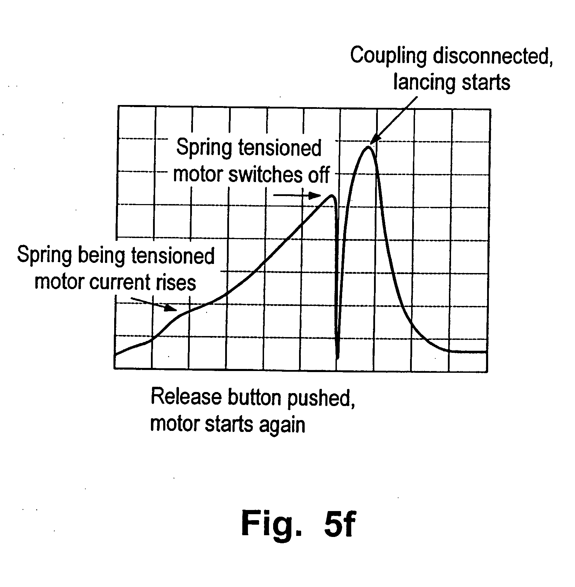

- Figur 5: System mit drehmomentgesteuerter Kupplung

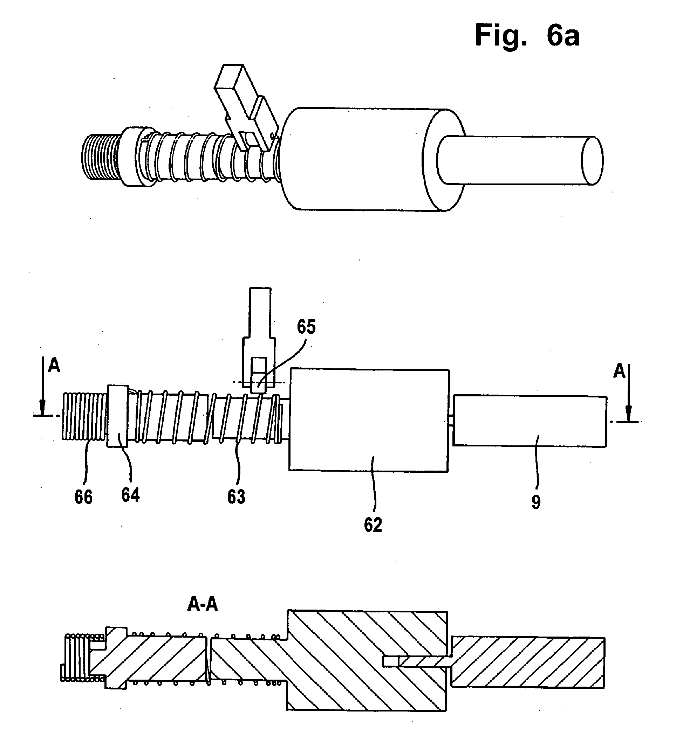

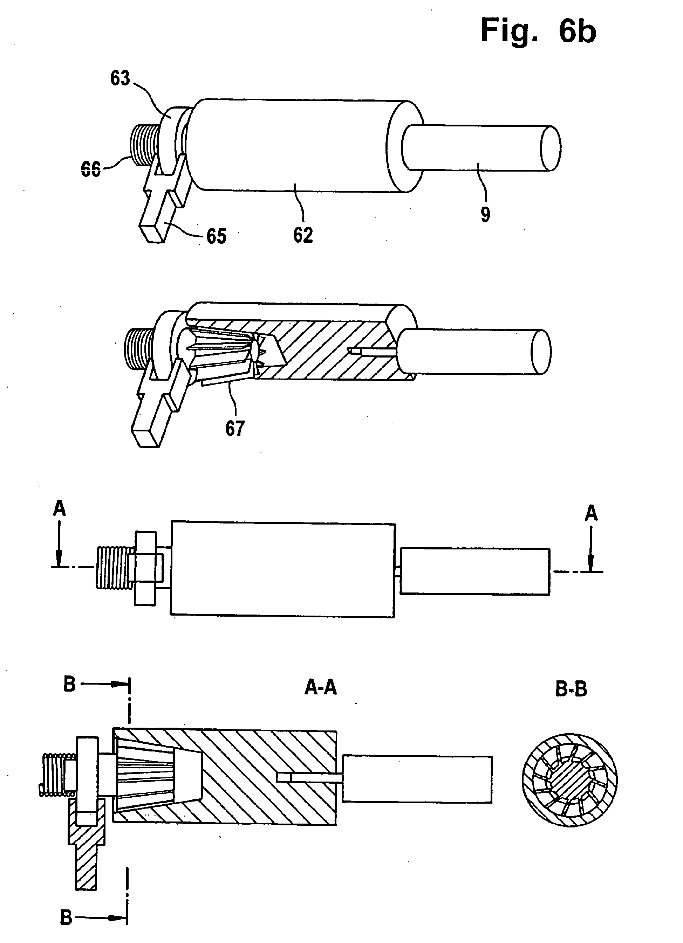

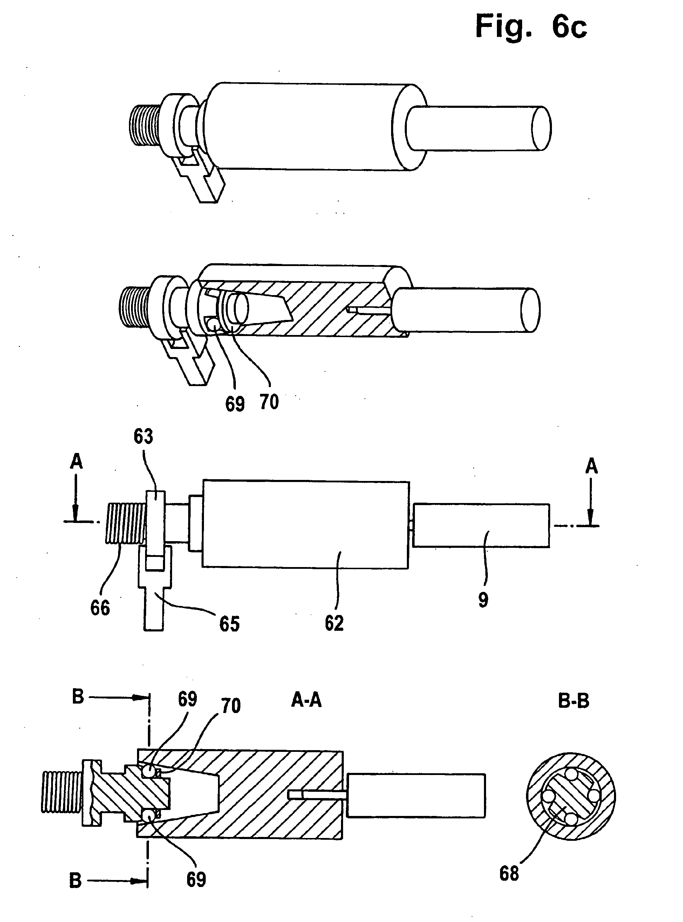

- Figur 6: System mit einer rotierenden Masse als mechanischer Energiespeicher.

Figur 1 a) zeigt eine Schnittdarstellung eines Blutentnahmegerätes (1). Das Gerät beinhaltet ein äußeres Gehäuse (2), das an seinem vorderen Ende (3) eine Austrittsöffnung (4) zum Austreten einer Lanzettenspitze aufweist. Die Austrittsöffnung (4) ist in einer Kappe (5) der Stechhilfe integriert, die drehbar mit dem Gehäuse (1) verbunden ist. Durch Drehen der Kappe (5) um die Achse (A) kann die Austrittsweite einer Nadelspitze aus der Öffnung (4) verändert werden und somit die Einstichtiefe des Blutentnahmegerätes von dem Benutzer gewählt werden. In dem vorderen Bereich (6) des Blutentnahmegeräts ist weiterhin eine Lanzette, bevorzugt ein Lanzettenmagazin (nicht gezeigt) angeordnet, aus dem Lanzetten zum Einstichvorgang entnommen werden. Zum Ausführen des Stechvorgangs wird die Lanzette über eine Antriebseinheit (8) in Einstichrichtung entlang der Achse (A) angetrieben und nach dem Stechvorgang wieder in das Gehäuse zurückgezogen. In dem gezeigten Beispiel beinhaltet die Antriebseinheit einen Motor (9), der mit einem elektrischen Energiespeicher in Form einer Batterie verbunden ist (nicht gezeigt). Der elektrische Motor ist über ein Getriebe (10) an eine Kupplung (11) gekoppelt. Wird der Motor von dem Benutzer zum Ausführen eines Stechvorgangs aktiviert, wird eine Rotationsbewegung über Getriebe und Kupplung auf die Feder (12) übertragen, die auf diese Weise komprimiert wird. Hierbei wird über die Kupplung das für die Komprimierung der Feder notwendige Drehmoment generiert, so dass auch bei geringen Leistungen des Motors eine hinreichende Kompression der Feder erfolgen kann. Vorteilhafterweise erfolgt eine Steuerung des Motors über die Messung des anliegenden Motorstroms und somit des vorherrschenden Drehmomentes. Wird hierbei ein vorbestimmter Grenzwert überschritten, wird dem System signalisiert, dass die Feder nun hinreichend vorgespannt ist, wobei der Motor automatisch gestoppt wird. Über einen Auslöseknopf (7) kann der Benutzer nun den Stechvorgang auslösen. Hierbei aktiviert der Benutzer erneut durch Betätigung des Auslöseknopfes den Motor, wobei die Feder erneut komprimiert wird, bis ein zweites vorbestimmtes Drehmoment erreicht wird. Bei Erreichung des zweiten Grenzwertes erfolgt eine automatische Loslösung der zuvor im gespannten Zustand arretierten Feder, so dass die von der Feder gespeicherten potentielle Energie freigesetzt werden kann. Die freiwerdende Energie der Feder wird nun über einen Bewegungswandler (nicht gezeigt) z. B. eine Steuerkulisse auf den Lanzettenkörper umgelenkt, so dass die Lanzette eine zwangsläufig geführte Bewegung ausführt und ein schmerzarmer Einstich in ein Körperteil ausgeführt werden kann.

Figur 1 b) skizziert ein Grobfunktionsmuster einer automatisierten Stechhilfe und verdeutlicht dabei, die Ankopplung des Motors an einen mechanischen Energiespeicher sowie an einen mechanische Bewegungswandler, der die Einstichbewegung des Lanzettenkörpers führt. In dem gezeigten Beispiel ist der Motor (9) lediglich schematisch verdeutlicht und über ein Getriebe (10) sowie eine Kupplung (11) mit einer Feder (20) als mechanischer Energiespeicher gekoppelt. Die Feder ist an ihrem zweiten Ende mit einer Kulisse (15) verbunden, die in einem erfindungsgemäßen System über die Lager (13) beweglich gelagert wird. Die Kulisse weist eine Führungsnut (16) auf, die als Steuerkulisse für den Lanzettenhalter (14) dient. Wird der Motor zum Spannen der Feder aktiviert, erfolgt eine Kompression der Feder, wobei die Kulisse an den Motor herangezogen wird. Die Kulisse ist folglich lateral zum Motor verschiebbar. Der Lanzettenhalter (14) ist hingegen entlang der Richtung (B) ortsfest im System positioniert und verbleibt in seiner lateralen Position relativ zum Motor ortsfest, während die Kulisse eine seitliche Verschiebung erfährt. Der Lanzettenhalter wird währenddessen entlang der Führungsnut (16) geführt, die als Steuerkurve eine Auslenkung des Lanzettenhalters senkrecht zur Bewegung der Kulisse bedingt. Der Lanzettenhalter erfährt folglich einen Bewegungshub entlang der Einstichrichtung (A) und wird anschließend durch die Ausbildung der Führungsnut in seine ursprüngliche Position wieder zurückgeholt. Nach dem Spannen wird die Kulisse ortsfest im System arretiert. Beim Auslösen des Stechvorgangs wird zunächst die Arretierung gelöst, so dass die Feder wieder in ihren entspannten Zustand zurückkehren kann. Aufgrund der daraus resultierenden Bewegung der Kulisse senkrecht zur Einstichrichtung (A) durchläuft der Lanzettenhalter (14) erneut die Führungsnut (16) wobei der Stechvorgang ausgeführt wird.

Figur lc zeigt ein erfindungsgemäßes System mit einem Kreuzschleifgetriebe als mechanischen Bewegungswandler. Das System weist einen elektrisch angetriebenen Motor(9) auf, der über Lager (19) ortsfest im System positioniert ist. Der Motor ist mit einem Kegelradgetriebe (10) verbunden, welches wiederum an eine Kupplung (11) (nur schematisch angedeutet) gekoppelt ist. Aufgrund des Kegelradgetriebes ist es möglich, den räumlichen Aufbau des Systems flexibel zu gestalten, so dass der Motor, wie beispielhaft in der Abbildung gezeigt, nicht linear hinter dem Lanzettenhalter (14) angeordnet sein muss. Eine Umlenkung der von dem Motor zur Verfügung gestellten Energie um 90° wird somit problemlos möglich. Auf diese Weise kann eine kompakte Bauweise des Systems erzielt werden. Ebenso ist die Integration von weiteren Systemfunktionen (hier nicht gezeigt) möglich, wobei der räumliche Aufbau des erfindungsgemäßen Systems entsprechend an die weiteren Systemfunktionen angepasst werden kann. Wird das Kegelradgetriebe und die Kupplung über den Motor angetrieben, erfolgt das Spannen einer Spiralfeder (12). Im beschriebenen Beispiel erfolgt eine drehwinkelabhängige Steuerung des Motors, wobei der Motor gestoppt wird, sobald eine Drehung der Kupplung um 360° erfolg ist. Die Feder wird in ihrem gespannten Zustand arretiert. Das Blutentnahmesystem liegt nun betriebsbereit vor. Durch Betätigung eines Auslöseschalters (7) wird die Feder freigegeben und die gespeicherte potentielle Energie wird über ein Kreuzschleifgetriebe (18) auf den Lanzettenkörper übertragen. Im gezeigten Beispiel, wird als mechanischer Bewegungswandler eine Kreuzschleifgetriebe verwendet, das eine zwangsläufig geführte Bewegung des Lanzettenhalters bedingt. Die beschriebene Ausführungsform zeigt beispielhaft eine Kombination von verschiedenen möglichen Bauelementen eines erfindungsgemäßen Systems. Ebenso ist es auch denkbar, dass anstelle eines Kreuzschleifgetriebes eine Kulissenführung, wie in Figur 1b beschrieben, in das System integriert wird. Hierbei erlaubt eine vielseitige Kombination der einzelnen Bauelemente eine flexible Ausgestaltung eines erfindungsgemäßen Systems, das entsprechend den Anforderungen insbesondere für integrierte Systeme angepasst werden kann.

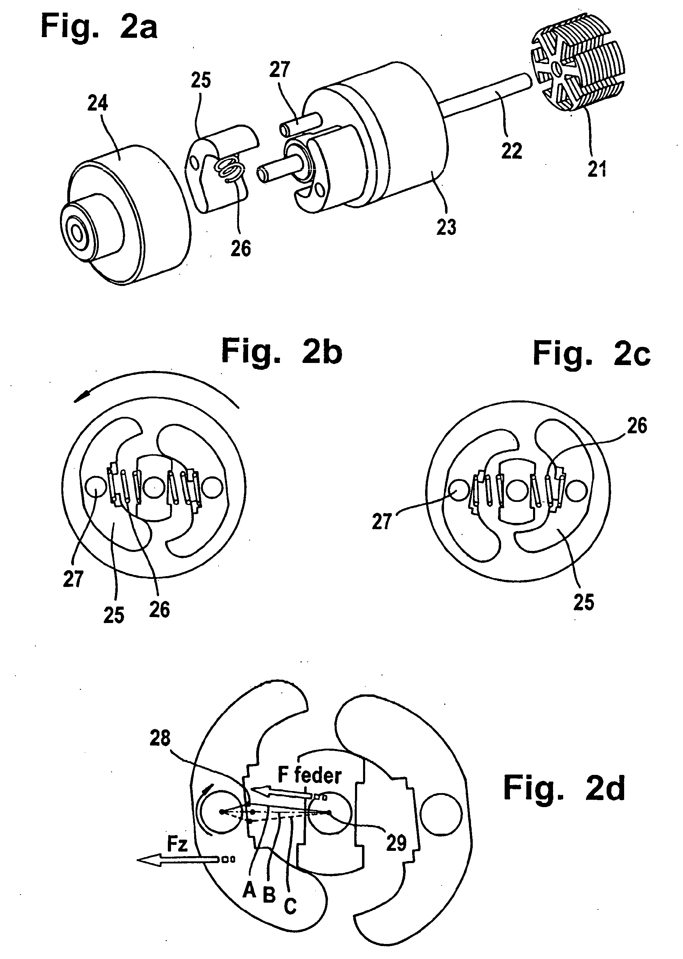

Figur 2 zeigt eine detaillierte Ansicht eines Antriebes, bei dem als mechanischer Energiespeicher eine rotierende Masse verwendet wird. Im wesentlichen ist der Aufbau eines derartigen Systems analog zu dem bereits in Figur 1 dargestellten Blutentnahmegerätes. Anstelle des mechanischen Energiespeichers, der in Figur 1 durch eine Feder dargestellt wird, wird jedoch hier eine rotierende Masse verwendet. Hierdurch ergeben sich einige Anpassungen im System, so dass eine schnelle und effiziente Energieübertragung der rotierenden Masse, auf den Lanzettenkörper erfolgen kann. Im folgenden wird ausschließlich die Detailansicht für die Systemkomplexe gezeigt, die eine unmittelbare Energieübertragung einer rotierenden Masse auf einen Lanzettenkörper ermöglichen. Figur 2 verdeutlicht hierbei die Funktionsweise einer Automatikkupplung, die die kinetische Energie sowohl direkt auf einen Lanzettenkörper überträgt oder wiederum zunächst an eine Feder gekoppelt ist, sodass eine indirekte Ankopplung der Kupplung an den Lanzettenkörper über eine Feder erfolgt. Wird die Kupplung an eine Feder gekoppelt, erfolgt hierdurch zunächst eine Umwandlung der kinetischen Energie in potentiell Energie, die in der Feder zunächst zwischengespeichert wird. Hierbei wird aufgrund eines schlagartigen Einkuppelns der Automatikkupplung hinreichend Energie der rotierenden Masse zum Spannen der Feder auf diese übertragen. Natürlich ist auch eine direkte Verbindung der Automatikkupplung an den Lanzettenkörper möglich. Vor- bzw. Nachteile dieser Systemvarianten ergeben sich wie nachfolgend in Figur 6 beschrieben. Analog zu dem in Figur 1 gezeigten System erfolgt darüberhinaus eine Ankopplung der Bauelemente an einen Motor, Lanzettenkörper etc., die zur Vereinfachung der Darstellung hier nicht gezeigt werden.

Figur 2 a) zeigt eine Explosionsansicht eines Antriebes mit einer rotierenden Masse sowie eine Automatikkupplung. Als Antrieb wird dabei ein bürstenloser Außenläufermotor verwendet. Er besteht im Wesentlichen aus einem Statorblechpaket (21) mit aufgebrachter Wicklung (nicht dargestellte einem Weicheisenrotor (23) mit eingebauten Magneten und einer gemeinsamen Welle (22). Fest mit dem Rotor (23) verbunden ist eine Automatikkupplung, betstehend aus den Bauelementen 24-27. Eine Kupplungsglocke (24) ist wie oben beschrieben entweder direkt mit einem anzutreibenden Stechgetriebe oder über eine Feder mit diesen axial verbunden (nicht dargestellt). Das Statorpaket (21) und die Welle (22) sind ortsfest (nicht rotierend) im System positioniert. Im eingeschalteten Zustand rotiert der Rotor (23), wobei die mit dem Rotor fest verbundenen Kupplungselemente (25-27) dem Bewegungsablauf folgen und um den Stator (21) rotieren. Die Kupplungsglocke (24) ist drehbar auf der gemeinsamen Welle (22) gelagert und ist mit den Bauelementen (23, 25, 26, 27) nicht verbunden, so dass die Kupplungsglocke zunächst ortsfest im System verbleibt. Oberhalb einer Grenzdrehzahl werden die rotierenden Bauelemente der Automatikkupplung schlagartig mit der Kupplungsglocke (24), verkuppelt. Die gespeicherte Rotationsenergie des Rotors sowie der rotierenden Bauelementen wird damit in die Kupplungsglocke und das hiermit verbundene Stechgetriebe oder eine Feder überführt. Nach dem Kupplungsvorgang ist der Motor blockiert und wird von einer Steuerelektronik abgeschaltet. Die Automatikkupplung trennt anschließend selbsttätig die Verbindung zwischen Rotor (23) und Kupplungsglocke (24). Das System ist für einen erneuten Vorgang bereit.

Die detaillierte Funktionsweise der Automatikkupplung ist in den Abbildungen 2b - 2d dargestellt. Abb. 2b zeigt die Kupplung im ausgekuppelten Zustand unterhalb der Grenzdrehzahl. Die beiden symmetrisch angeordneten Kupplungsbacken (25) werden durch die Federn (26) in einer Ruhelage gehalten. Die Oberflächen der Kupplungsbacken (25) haben keinen Kontakt zu der umgebenden Kupplungsglocke (nicht dargestellt). Mit überschreiten der Grenzdrehzahl drehen sich die Kupplungsbacken (25) um die Lagerstifte (27) und berühren die Kupplungsglocke. Durch Reibschluss der Backen (25) mit der Innenwandung der Glocke erfolgt das Einkuppeln von der Kupplung in die Kupplungsglocke (24), so dass die Kupplungsglocke der Rotationsbewegung folgt. Zur Vermeidung von Reibungsverlusten während des Einkupplungsvorgangs muss dieser möglichst schlagartig ablaufen. Dazu ist eine spezielle Federanordnung (26) als Sprungwerk wie nachfolgend erläutert gewählt. In Abbildung 2d ist die Lage der Feder schematisch während des Einkupplungsvorgangs anhand der Linien A-C dargestellt. Dabei symbolisiert die Linie A die Ausgangslage (nicht eingekoppelt) der Kupplung, während die Linie C die Endlage der Feder oberhalb der Grenzdrehzahl darstellt. Die Feder ist zwischen den Punkten (28) und (29) im System befestigt und arbeitet als Druckfeder. Wegen der Lage des Massenschwerpunktes der Kupplungsbacken außerhalb des Drehlagers entsteht bei der Rotation eine Zentrifugalkraft F_{z} proportional zur Drehzahl. Durch die gewählte Anordnung wird die Feder mit steigender Zentrifugalkraft F_{z} komprimiert. Das Maximum der Komprimierung wird mit der Grenzdrehzahl erreicht und wird durch die Line B symbolisiert. Dieser Zustand ist jedoch instabil und führt direkt und schlagartig zum Weiterdrehen der Backen in die Position entsprechend der Linie C. Sobald der Rotor gestoppt wird, erfolgt ein Auskuppeln der Kupplungsglocke. Hierbei reduziert sich durch das Anhalten des Motors die Zentrifugalkraft F_{z} auf Null, wobei aufgrund der Federkraft F_{feder} die Kupplungsbacken wieder in die ausgekuppelte Ausgangslage gedreht werden. Die beschriebene Ausführungsform einer Automatikkupplung gewährleistet, dass durch das Einkuppeln der Kupplungsglocke die Energie schlagartig auf die Lanzettenhalter direkt oder indirekt übertragen werden kann. Hierdurch wird eine hinreichend schnelle Entnahmegeschwindigkeit der Energie ermöglicht, so dass eine Einstichvorgang des Lanzettenkörpers schmerzarm durchgeführt werden kann, bzw. das Spannen einer Feder als Zwischenspeicher möglich ist.

Figur 3 a) zeigt schematisch einen möglichen Aufbau eines kombinierten Antriebes (Kombiantrieb). Aufgrund eines erfindungsgemäßen Kombiantriebes wird eine weitere Miniaturisierung sowie Verringerung des Gerätegewichtes für integrierte Systeme möglich. Dem Benutzer wird somit bei einem kompakten, tragbaren Geräten ein bequemes Handling gewährleistet. Des weiteren werden die Möglichkeiten einer Fehlbedienung des Systems reduziert. Hierbei ist der Motor (9) über ein Getriebe (10) mit einem Zahnrad (32) verbunden, welches drehbar im System gelagert ist. Durch Betätigung des Motors kann das Zahnrad eine Rotationsbewegung in unterschiedliche Drehrichtungen ausführen. Das Zahnrad ist im gezeigten Beispiel sowohl mit einer Feder (20) zum Speichern von mechanischer Energie gekoppelt als auch direkt mit einer Gehäuseseite eines Trommelmagazins (34). Der Motor ist folglich über ein Getriebe (10) an zwei Systemfunktionen gekoppelt. Erfolgt eine Rotation des Zahnrades wird hierdurch zum einen die Feder (20) komprimiert. Zum anderen greift das Zahnrad in einen entsprechend ausgestalteten Boden eines Trommelmagazins ein, so dass das Magazin um seine Längsachse gedreht wird. Das Magazin kann beispielsweise zur Magazinierung von Teststreifen oder Lanzetten vorgesehen sein, so dass eine Rotation das Magazin in der Weise erfolgt, dass ein Disposable im Magazin entsprechend zu einer Entnahmeeinheit in dem Gerät positioniert wird. So ist es z. B. denkbar, dass während des Spannens der Feder zum Antreiben einer Lanzette zeitgleich ein Weitertakten der Trommel erfolgt, so dass ein Teststreifen aus dem Magazin zur Probenaufgabe mittels einer hierfür vorgesehenen Entnahmeeinheit z.B. eines Stößels entnommen werden kann.

Figur 3 b) zeigt eine detaillierte Ansicht des in Figur 3 a) gezeigten Kombiantriebs, der die Funktion eines Magazintransporters sowie das Spannen einer Feder zum Antreiben einer Lanzette leistet. Der Kombiantrieb besteht aus einem DC-Motor (9), der gleichzeitig das Weitertakten eines Trommelmagazins (nicht gezeigt) und das Spannen einer Stechhilfe (35) leistet. Der Motor ist für den Trommelantrieb mit einem Getriebe (36) verbunden. Das Getriebe leitet die im Motor zur Verfügung gestellte elektrische Energie auf eine Welle (37) um, die in Rotation versetzt wird. Die Welle weist an ihrem oberen Kopf eine zahnartige Struktur auf, die in einem entsprechend ausgeformten Gehäuseboden eines Trommelmagazins (nicht gezeigt) eingreift. Ein Trommelmagazin wird somit beim Einlegen in das Messgeräte auf die Well (37) aufgesetzt und dort arretiert. Wird der Motor aktiviert und die Welle in Rotation versetzt, folgt die Trommel der Bewegung. Um ebenfalls Energie für das Spannen der Stechhilfe abzugreifen, ist weiterhin ein Stirnrad (40) mit dem Getriebe (36) verbunden. Im gezeigten Beispiel erfolgt somit ein Weitertakten des Magazins während gleichzeitig die Feder des Blutentnahmesystems gespannt wird. Der Benutzer kann nun auf Wunsch einen Stechvorgang auslösen und die Ausgabe eines neuen Testelementes aus dem Magazin anfordern. Es ist natürlich denkbar, die Systemfunktionen (Spannen der Feder und Weitertakten des Magazins) zeitlich voneinander zu trennen. Unter diesen Umständen beinhaltet das System vorteilhafterweise eine Kupplung, die eine Systemfunktion von dem Motor entkoppelt bzw. ankoppelt, sofern eine Aktivierung der jeweiligen Systemfunktion gewünscht ist. Der Aufbau der Stechhilfe ist zunächst beliebig gewählt, wobei im gezeigten Beispiel als mechanischer Energiespeicher eine Spiralfeder verwendet wird. Für den weiteren Aufbau der Stechhilfe wird an dieser Stelle voll inhaltlich Bezug auf bereits im Stand der Technik bekannte Systeme, beispielsweise DE 10336933.3, genommen.

Prinzipiell ist die Verwendung eines erfindungsgemäßen Kombiantriebes für beliebige Systemfunktionen denkbar und nicht auf bestimmte Anwendungen beschränkt. Beispielhaft sei hier ein Testelementtransport etc. genannt.

Figur 4 zeigt eine Detailansicht eine drehwinkelgesteuerte Kupplung wie sie beispielsweise zur Kopplung des elektrischen Motors mit einer Feder Anwendung findet. Die Kupplung ist an einem ersten Wellenbereich (45) einer Welle (47) mit einer Feder (nicht gezeigt) einer Stechhilfe kontaktiert, während ein zweiter Wellenbereich (46) der Welle mit einem elektrischen Motor (nicht gezeigt) in Verbindung steht und von diesem angetrieben wird. Hierfür wird der Wellenbereich (46) in eine Schnittstelle des Motors (nicht gezeigt)eingeführt und von diesem gedreht. Der Wellenbereich (46) weist an seinem, nicht mit dem Motor verbundenen Ende, eine Zahnstruktur (42) auf, die in die Zahnstruktur des gegenüberliegenden Endes (41) des Wellenbereiches (45) eingreift. Aufgrund des drehsicheren Ineinandergreifens der beiden Wellenenden wird gewährleistet, dass eine durch den Motor verursachte Drehbewegung des Wellenbereiches (46) auf den Wellenbereich (45) übertragen wird. Eine Drehung des Wellenbereiches (46) hat somit eine Drehbewegung der gesamten Welle (47) zur Folge. Eine mit dem Wellenbereich (45) fest verbundene Feder (nicht gezeigt) erfährt so infolge der Drehung der Welle eine Komprimierung und wird hierdurch gespannt. Die Kupplung weist weiterhin eine Kulisse (44) auf, in der der erste Wellenbereich (46) durch einen Bolzen (43), der fest mit dem Wellenbereich (46) verbunden ist, geführt wird. Erfährt der Wellenbereich (46) eine Drehung, wird der Bolzen (43) entsprechend entlang der Kulisse (44) geführt. Hierdurch wird der Bolzen (43) und der hiermit fest verbundene Wellenbereich (46) entsprechend der Kontur der Kulisse axial ausgelenkt. Auf grund der Axialverschiebung des Wellenbereiches (46) kommt es zum Entkoppeln der Zahnstrukturen (41) und (42) sodass die Wellenbereiche (45) und (46) voneinander losgelöst werden. Aufgrund des durch die Kompression der Feder wirkenden Drehmomentes, erfolgt nun eine Rotation des Wellenbereiches (45) in entgegengesetzter Richtung, so dass der Federantrieb nahezu reibungsfrei zurücklaufen kann. Die so freigesetzte Energie wird auf einen Lanzettenkörper übertragen, so dass der Lanzettenkörper in Einstichrichtung bewegt wird. Figur 4 zeigt somit eine Kupplung, die in Abhängigkeit von einem festgelegten Drehwinkel, der durch die Kontur der Kulisse vorbestimmt wird, eine Steuerung des Motors und somit der Antriebseinheit erlaubt. Durch Drehen der Welle (47) erreicht der Bolzen (43) des Wellenbereichs (46) zunächst eine Position (48) der Kulisse. In dieser Position erfährt der Wellenbereich (46) eine erste axiale Auslenkung wodurch der Motor gestoppt wird. Zum Auslösen des Stiches wird der Motor durch den Benutzer erneut aktiviert. Mit zunehmendem Verdrehwinkel folgt der Bolzen (43) weiterhin der Zwangsführung durch die Kulisse bis er die Position (49) erreicht. Hierdurch wird der Wellenbereich (46) in dem Maße ausgelenkt, dass eine Entkopplung der Wellenenden wie oben beschrieben erzielt wird.

Figur 5 zeigt eine drehmomentgesteuerte Kupplung, die ebenfalls als Kopplungsmechanismus zwischen dem Motor und einer Feder Anwendung findet. Die drehmomentgesteuerte Kupplung besteht aus einem ersten Antriebselement (52), das eine Blattfeder (53) aufweist. Ein weiteres Antriebselement (51) mit Stiften (54) ist drehbar mit dem Antriebselement (52) verbunden. Wie im Bild 5a) bis d) gezeigt, erfolgt ein Spannen der Feder in dem zunächst mittels eines Motors (nicht gezeigt) das Antriebselement (51) gedreht wird während das Antriebselement (52) ortsfest im System verbleibt. Hierdurch wird die Blattfeder (53) gegen die Stifte (54) gedrückt und somit verbogen. Mit zunehmendem Drehwinkel steigt das notwendig Drehmoment und somit der Motorstrom zum Spannen der Feder wie in der Graphik 5 f) veranschaulicht wird. Der Motorstrom wird von einer Antriebselektronik (nicht dargestellt) gemessen und mit einem eingestellten Grenzwert verglichen. Mit Erreichen eines ersten festgelegten Grenzwertes wird der Motor angehalten. Die Feder ist nun fertig gespannt (s. Figur 5 d). Zum Auslösen des Stechvorganges wird der Motor wieder eingeschaltet. Das Drehmoment steigt erneut an. Mit Erreichen des Auslösemomentes eines zweiten vergebenen Grenzwertes der Kupplung, welches größer als der erste eingestellte Grenzwert ist, löst sich die formschlüssige Verbindung zwischen der Blattfeder (53) und den Stiften (54) und die Feder kann nahezu reibungsfrei zurücklaufen. Die freigesetzte Energie wird zum Ausführen einer Stechbewegung umgesetzt (s. Figur 5 e)). Das angelegte Drehmoment fällt entsprechend wie in der Graphik (5 f) dargestellt, auf nahezu null zurück.

Figur 6 zeigt mehrere Ausführungsformen einer Kupplung wobei als mechanischer Energiespeicher eine in Rotation versetzte Masse verwendet wird. Auf diese Weise wird die für einen Stechvorgang benötigte Energie in Form von kinetischer Energie gespeichert und anschließend für einen Stechvorgang zur Verfügung gestellt. Bei der Verwendung einer Masse als mechanischer Energiespeicher, können u. a. auf Getriebe und/oder Kupplungen zur Kopplung des Motors an dem mechanischen Energiespeicher verzichtet werden. Hierdurch wird der Aufbau des Systems im Vergleich zu einem Aufbau mit einer Feder als mechanischen Energiespeicher vereinfacht. Ebenso kann eine bauliche Verkleinerung der Antriebseinheit erreicht werden. Wird eine bewegte Masse als mechanischer Energiespeicher verwendet, kann somit beispielsweise ein Elektromotor direkt mit der zu beschleunigenden Masse verbunden werden und diese in Rotation versetzen. Auf diese Weise wird zunächst durch eine einfache Bauweise kinetische Energie gespeichert. Die gespeicherte Energie muss jedoch zum Ausführen eine Stechvorganges schnell und verlustarm von dem Energiespeicher auf den Lanzettenkörper übertragen werden. Eine derartig Energieübertragung kann über eine geeignete Kupplung zwischen mechanischen Energiespeicher - hier die bewegte Masse - und der Lanzette erfolgen. Hierbei sollte die Kupplung möglichst verlustfrei arbeiten und kleine Schaltzeiten aufweisen, so dass Reibungsenergieverluste minimiert werden. Prinzipiell sind zwei vorteilhafte Ausführungsformen möglich, die eine Übertragung der Energie auf den Lanzettenkörper schnell und verlustarm zulassen. Zum einen kann eine direkte Umsetzung der kinetischen Energie auf den Lanzettenkörper oder auf einen mechanischen Bewegungswandler und somit in einen Stechvorgang erfolgen. Es ist jedoch auch denkbar, dass zunächst die Übertragung der Rotationsenergie auf einen weiteren Zwischenspeicher, der z. B. in Form einer Feder realisiert ist, erfolgt. Dies hat den Vorteil, dass beim Auslösen eines Stechvorganges nicht zunächst die Masse in Rotation versetzt werden muss, um die notwendige Energie für den Stechvorgang bereitzustellen. Ein derartiger Mechanismus würde zur Folge haben, dass beim Auslösen des Stechvorganges der Benutzer zunächst einige Sekunden warten müsste, bis die Masse durch den Elektromotor entsprechend heschleunigt wird und anschließend eine Energieübertragung stattfinden kann. Wird jedoch ein Zwischenspeicher in Verbindung mit einer rotierenden Masse verwendet, ist es möglich, die durch die Rotation gewonnene Energie zunächst in einer Feder zwischenzuspeichern. Ein Auslösen des Stechvorganges durch z. B. Entspannen einer komprimierten Feder als Zwischenspeicher kann dann jederzeit und unmittelbar erfolgen und wird analog zu den bereits oben beschriebenen Systemen, bei dem eine Feder als mechanischer Energiespeicher verwendet wird, realisiert. Wird folglich eine rotierende Masse in Kombination mit einem Zwischenspeicher verwendet, kann dem Benutzer die gleichen Handhabungsmöglichkeiten, des erfindungsgemäßen Systems wie bereits beschrieben, geboten werden. Die rotierende Masse stellt somit in diesem Beispiel eine alternative Lösung zum Spannen einer Feder dar, ohne dass hierfür entsprechende Getriebe und/oder Kupplungen zur Verbindung von Motor und Energiespeicher notwendig sind. Ein Spannen einer Feder für das ansonsten hohe Drehmomente benötigt werden, ist somit durch eine unmittelbare Übertragung der kinetischen Energie auf die Feder möglich. Generell ermöglichst eine Kupplung zwischen der Masse und einem Zwischenspeicher, bzw. einem Lanzettenkörper, dass eine Übertragung der kinetischen Energie schlagartig in ca. 1 ms erfolgen kann. Dies erlaubt somit entweder eine direkte Umsetzung der Rotationsenergie in eine Stechbewegung oder ein effizientes Speichern der Energie in einen Zwischenspeicher trotz eines einfachen Aufbaus des Systems.

Figur 6 a zeigt einen Elektromotor (9), der zur Beschleunigung einer Masse (62) mit dieser verbunden ist und diese in Rotation versetzt. Durch drücken der Schalttaste (65) werden die Windungen einer Schlingfeder (63) auf den Zapfen der Rotationsmasse (62) gedrückt. Wird die Masse durch den Motor angetrieben, wickelt sich hierdurch die Feder (63) schlagartig auf den Zapfen auf, wodurch die Welle (64) eine Beschleunigung erfährt. In Abhängigkeit von dem verwendeten Antriebsprinzip wird die Rotationsbewegung der Welle (64) zum Spannen der Antriebsfeder (66), die als Zwischenspeicher verwendet wird benutzt, oder direkt in die Stechbewegung umgesetzt. Durch drücken der Schalttaste (65) kann somit zum einen direkt der Stechvorgang ausgelöst werden. Wird jedoch ein Zwischenspeicher (66) verwendet, wird die kinetische Energie durch automatische Betätigung der Schalttaste zunächst zwischengespeichert, wobei die Feder (66) komprimiert wird. Durch einen separaten Auslösevorgang für den Zwischenspeicher wird anschließend die Feder entspannt, wodurch ein Lanzettenkörper angetrieben werden kann. Um ein Ablösen einer gegebenenfalls verklemmten Schlingenfeder (63) zu erleichtern, kann der Motor nach Ausführen des Spann- bzw. des Stechvorganges kurz in entgegen gesetzte Richtung gedreht werden. Prinzipiell können für den Stechvorgang notwendige Verfahrensschritte automatisiert werden, so dass z. B. bei Erreichen einer vorgegebenen Rotationsfrequenz automatisch die Schalttaste (65) betätigt wird. Des weiteren kann das Ablösen der Schlingfeder und somit die Betätigung des Motors in entgegengesetzte Richtung automatisch nach Beendigung des Stechvorgangs initiiert werden. Häufig ist es jedoch gewünscht, dass das Auslösen des Stechvorgangs bewusst durch den Benutzer erfolgt.

Figur 6 b) zeigt ein weiteres Prinzip mit einer Reibrichtsperre, das eine Übertragung der kinetischen Energie einer rotierenden Masse auf einen Lanzettenkörper ermöglicht. Analog zu Figur 6 a), weist der mechanische Energiespeicher in Figur 6 b) zunächst einen Elektromotor (9) auf, der eine Masse (62) beschleunigt. Durch Schieben einer Schalttaste (65) erfolgt eine axiale Verschiebung der Reibrichtsperre (67). Hierdurch werden die Klemmstege der Reibrichtsperre (67) an die Außenwand des Konus der Rotationsmasse (62) gedrückt. Findet eine Verklemmung der Klemmstege mit der Masse (62) statt, wird die Reibrichtsperre schlagartig beschleunigt und folgt dem Bewegungslauf der Rotationsmasse (62). Analog zu den bereits oben beschriebenen Ausführungsformen, kann das Antriebsprinzip direkt an den Lanzettenkörper oder an einen Zwischenspeicher gekoppelt werden. Zum Lösen der Kupplungsverbindung und somit der Klemmverbindung zwischen der Reibrichtsperre (67) und der Rotationsmasse (62) wird die Reibrichtsperre (67) durch Schieben der Schalttaste (65) aus dem Konus der Rotationsmasse (62) herausgezogen.

Figur 6 c) zeigt eine weitere Ausführungsform zur Ankopplung einer rotierenden Masse an einen Lanzettenkörper bzw. Zwischenspeicher, wie er bereits in Figur 6 a und 6 b, vorgestellt wurde. Analog zu den oben beschriebenen Ausführungsformen weist das System einen Elektromotor (9) auf, der eine Masse (62) rotatorisch beschleunigt. Durch Schieben einer Schalttaste (65) wird ein schlagartiges Einkuppeln der Freilaufwelle (68) mit der Rotationsmasse (62) erreicht, so dass die kinetische Energie der Masse (62) möglichst verlustarm und direkt auf den Lanzettenkörper oder einem Zwischenspeicher übertragen werden kann. Das System weist hierfür eine Anzahl von Klemmkugeln (69) auf, die analog zu dem in Figur 6b vorgestellten Prinzips durch Schieben der Schalttaste mit dem Konus der Rotationsmasse (62) verklemmt werden. Hierbei verhindern Haltescheiben (70) das Herausfallen der Kugeln (69) durch die die Welle (63) an die Rotationsmasse (62) angekoppelt wird. Auf diese Weise wird die Freilaufwelle (68) gezwungen, der Bewegung der Rotationsmasse (62) zu folgen, wobei die Energie innerhalb einer Millisekunde von der Rotationsmasse auf die Welle (69) übertragen wird. Zum Lösen der Kupplungsverbindung wird auch hier durch Schieben der Schalttaste (65) die Kupplungsverbindung zwischen der Welle (64) und der Rotationsmasse (62) gelöst, indem die Welle (64) aus dem Konus der Masse herausgezogen wird.

## Patentansprüche

1. Stechhilfe zum Erzeugen einer Hautöffnung in einem Körperteil beinhaltend
- ein Gehäuse (2) mit einer Öffnung (4), aus der eine Lanzette austreten kann,
- eine Lanzette mit einem Lanzettenkörper, sowie
- eine Antriebseinheit (8) zum Antreiben des Lanzettenkörpers und der Lanzette, so dass die Lanzettespitze der Lanzette zum Ausführen eines Stechvorgangs zumindest zum Teil aus dem Gehäuse (2) austreten kann, wobei
- die Antriebseinheit (8) beinhaltet
- einen Motor (9), der mit einem elektrischen Energiespeicher kontaktiert werden kann, sodass Energie zum Antreiben der Lanzette bereitgestellt wird,
- einen mechanischen Energiespeicher, der an den Motor in der Weise gekoppelt ist, dass die von einem elektrischen Energiespeicher gespeicherte elektrische Energie in mechanische Energie umgewandelt und vom mechanischen Energiespeicher zumindest zum Teil gespeichert wird, sowie
- einen Kopplungsmechanismus, der den Lanzettenkörper an den mechanischen Energiespeicher ankoppelt, so dass die gespeicherte Energie des Energiespeichers auf den Lanzettenkörper zumindest zum Teil übertragen werden kann, wobei
- der Kopplungsmechanismus einen mechanischen Bewegungswandler beinhaltet, der die Energie des Energiespeichers in der Weise auf den Lawzettenkörper-umlenkt, dass eine Steuerung der Lanzettenbewegung in eine zwangsläufig geführte Bewegung überführt wird, **dadurch gekennzeichnet, dass**
- die Stechhilfe (1) in einem Messgerät zur Bestimmung eines Analyten aus einer Blutprobe integriert ist und
- der Motor (9) an eine weitere von dem mechanischen Energiespeicher unabhängige Systemfunktion im Messgerät gekoppelt ist.

2. Stechhilfe gemäß Anspruch 1, bei der der mechanische Energiespeicher als Festkörper in der Stechhilfe integriert ist.

3. Stechhilfe gemäß Anspruch 1, bei der der mechanische Bewegungswandler eine Steuerkulisse (15) aufweist.

4. Stechhilfe gemäß Anspruch 1, bei der der mechanische Bewegungswandler ein Kreuzschleifgetriebe aufweist.

5. Stechhilfe gemäß einer der Ansprüche 1 - 4, bei der der Motor (9) ein Getriebe (10) aufweist, durch das der Motor an den Energiespeicher gekoppelt wird.

6. Stechhilfe gemäß einer der Ansprüche 1 - 5, bei der der Motor eine Kupplung (11) aufweist, durch die der Motor (9) an den Energiespeicher gekoppelt wird.

7. Stechhilfe gemäß Anspruch 2, bei der der mechanische Energiespeicher eine Feder (12) ist.

8. Stechhilfe gemäß Anspruch 6, bei der der Motorstrom in der Antriebseinheit gemessen und mit vordefinierten Werten verglichen wird und eine Steuerung des Motors (9) auf der Basis dieses Vergleiches erfolgt.

9. Stechhilfe gemäß Anspruch 6, bei der die Kupplung (11) mindestens ein Drehmoment von 10 mNm auf den Energiespeicher überträgt.

10. Stechhilfe gemäß Anspruch 5, bei der das Getriebe (10) ein Kegelradgetriebe ist.

11. Stechhilfe gemäß Anspruch 2, bei der der mechanische Energiespeicher-eine-Masse (62) ist.

12. Stechhilfe gemäß Anspruch 1, bei der der Kopplungsmechanismus eine Kupplung (11) beinhaltet.

13. Stechhilfe gemäß Anspruch 6, bei der die Kupplung drehmoments- oder drehwinkelgesteuert ist.

14. Stechhilfe gemäß Anspruch 1, bei der der elektrische Motor (9) ein Piezomotor oder ein Außenläufermotor ist.

15. Stechhilfe gemäß Anspruch 1, bei der die Energie für den mechanischen Energiespeicher und die Energie für die von dem Energiespeicher unabhängige Systemfunktion gleichzeitig oder unabhängig voneinander bereitgestellt wird.

16. Stechhilfe gemäß Anspruch 1, bei der die von dem Energiespeicher unabhängige Systemfunktion ein Testelementtransport oder ein Magazintransport ist.

17. Stechhilfe gemäß Anspruch 1, bei der mehrere Testelemente in einem Magazin im Messgerät gelagert werden.

## Claims

1. Lancing aid for producing a skin opening in a body part comprising
- a housing (2) with an opening (4) from which a lancet can emerge,
- a lancet with a lancet body, as well as
- a drive unit (8) for driving the lancet body and the lancet such that the lancet tip of the lancet can at least partially emerge from the housing (2) in order to perform a lancing process, wherein
- the drive unit (8) comprises
- a motor (9) which can be contacted with an electrical energy store so that energy is provided to drive the lancet,
- a mechanical energy store which is coupled to the motor in such a manner that the electrical energy stored by an electrical energy store is converted into mechanical energy and is at least partially stored by the mechanical energy store, as well as
- a coupling mechanism which couples the lancet body to the mechanical energy store such that the stored energy of the energy store can be transferred at least partially onto the lancet body, wherein
- the coupling mechanism comprises a mechanical motion converter which diverts the energy of the energy store onto the lancet body in such a manner that a control of the lancet movement is converted into a positively guided movement, **characterized in that**
- the lancing aid (1) is integrated in a measuring device for determining an analyte from a blood sample and
- the motor (9) is coupled to a further system function in the measuring device which is independent of the mechanical energy store.

2. Lancing aid according to claim 1, in which the mechanical energy store is integrated as a solid body in the lancing aid.

3. Lancing aid according to claim 1, in which the mechanical motion converter has a control guide block (15).

4. Lancing aid according to claim 1, in which the mechanical motion converter has a cross-slip gear unit.

5. Lancing aid according to one of the claims 1 - 4 in which the motor (9) has a gear unit (10) by means of which the motor is coupled to the energy store.

6. Lancing aid according to one of the claims 1 - 5, in which the motor has a clutch (11) by means of which the motor (9) is coupled to the energy store.

7. Lancing aid according to claim 2, in which the mechanical energy store is a spring (12).

8. Lancing aid according to claim 6, in which the motor current in the drive unit is measured and compared with predefined values and the motor (9) is controlled on the basis of this comparison.

9. Lancing aid according to claim 6, in which the clutch (11) transfers at least a torque of 10 mNm onto the energy store.

10. Lancing aid according to claim 5, in which the gear unit (10) is a bevel gear unit.

11. Lancing aid according to claim 2, in which the mechanical energy store is a mass (62).

12. Lancing aid according to claim 1, in which the coupling mechanism comprises a clutch (11).

13. Lancing aid according to claim 6, in which the clutch is torque-controlled or controlled according to the angle of rotation.

14. Lancing aid according to claim 1, in which the electric motor (9) is a Piezo motor or a motor of the external rotor type.

15. Lancing aid according to claim 1, in which the energy for the mechanical energy store and the energy for the system function that is independent of the energy store is provided simultaneously or independently of one another.

16. Lancing aid according to claim 1, in which the system function that is independent of the energy store is a test element transport or a magazine transport.

17. Lancing aid according to claim 1, in which several test elements are stored in a magazine in the measuring device.

## Revendications

1. Aide à la perforation pour générer une ouverture de peau dans une partie de corps comprenant
- un boîtier (2) avec une ouverture (4), de laquelle une lancette peut sortir,
- une lancette avec un corps de lancette, et
- une unité d'entraînement (8) pour l'entraînement du corps de lancette et de la lancette, de sorte que la pointe de lancette peut sortir au moins en partie du boîtier (2) pour exécuter une opération de perforation,
- l'unité d'entraînement (8) contenant
- un moteur (9), qui peut être mis en contact avec un accumulateur d'énergie électrique, de sorte que de l'énergie est mise à disposition pour l'entraînement de la lancette,
- un accumulateur d'énergie mécanique, qui est couplé au moteur en ce sens que l'énergie électrique accumulée par un accumulateur d'énergie électrique est transformée en énergie mécanique et est stockée en moins en partie par l'accumulateur d'énergie mécanique, et
- un mécanisme d'accouplement, qui associe le corps de lancette à l'accumulateur d'énergie mécanique, de sorte que l'énergie stockée de l'accumulateur d'énergie peut être transmise au moins en partie au corps de lancette,
- le mécanisme d'accouplement contenant un convertisseur de mouvement mécanique, qui dévie l'énergie de l'accumulateur d'énergie sur le corps de lancette en ce sens qu'une commande du mouvement de lancette est convertie dans un mouvement guidé de force, **caractérisée en ce que**
- l'aide à la perforation (1) est intégrée dans un appareil de mesure pour déterminer un analyte à partir d'un échantillon de sang et
- le moteur (9) est couplé à une autre fonction de système, indépendant de l'accumulateur d'énergie mécanique, dans l'appareil de mesure.

2. Aide à la perforation selon la revendication 1, sur laquelle l'accumulateur d'énergie mécanique est intégré sous forme de corps solide dans l'aide à la perforation.

3. Aide à la perforation selon la revendication 1, sur laquelle le convertisseur de mouvement mécanique présente une coulisse de commande (15).

4. Aide à la perforation selon la revendication 1, sur laquelle le convertisseur de mouvement mécanique présente un engrenage à passes croisées.

5. Aide à la perforation selon l'une des revendications 1-4, sur laquelle le moteur (9) présente un engrenage (10), par lequel le moteur est couplé à l'accumulateur d'énergie.

6. Aide à la perforation selon l'une des revendications 1-5, sur laquelle le moteur présente un accouplement (11), par lequel le moteur (9) est couplé à l'accumulateur d'énergie.

7. Aide à la perforation selon la revendication 2, sur laquelle l'accumulateur d'énergie mécanique est un ressort (12).

8. Aide à la perforation selon la revendication 6, sur laquelle le courant de moteur est mesuré dans l'unité d'entraînement et est comparé avec des valeurs prédéfinies et l'on a une commande du moteur (9) sur la base de cette comparaison.

9. Aide à la perforation selon la revendication 6, sur laquelle l'accouplement (11) transmet au moins un couple de 10 mNm à l'accumulateur d'énergie.

10. Aide à la perforation selon la revendication 5, sur laquelle l'engrenage (10) est un engrenage conique.

11. Aide à la perforation selon la revendication 2, sur laquelle l'accumulateur d'énergie mécanique est une masse (62).

12. Aide à la perforation selon la revendication 1, sur laquelle le mécanisme d'accouplement contient un accouplement (11).

13. Aide à la perforation selon la revendication 6, sur laquelle l'accouplement est commandé par couple ou angle de rotation.

14. Aide à la perforation selon la revendication 1, sur laquelle le moteur électrique (9) est un moteur piézoélectrique ou un moteur à induit extérieur.

15. Aide à la perforation selon la revendication 1, sur laquelle l'énergie pour l'accumulateur d'énergie mécanique et l'énergie pour la fonction du système indépendante de l'accumulateur d'énergie sont mises à disposition en même temps ou indépendamment l'une de l'autre.

16. Aide à la perforation selon la revendication 1, sur laquelle la fonction du système indépendante de l'accumulateur d'énergie est un transport d'élément de test ou un transport de magasin.

17. Aide à la perforation selon la revendication 1, sur laquelle plusieurs éléments de test sont stockés dans un magasin dans l'appareil de mesure.
